# EUROPÄISCHE PATENTANMELDUNG

(11) **EP 1 356 762 A1**
(43) Veröffentlichungstag der Anmeldung: **29.10.2003**
(21) Anmeldenummer: 02008958.7
(22) Anmeldetag: 22.04.2002
(51) Int. Cl.: A61B 5/00, A61B 5/02, A61B 5/04, G06F 19/00

(54) **Geräteanordnung zur Fernüberwachung von Körperfunktionen**

(71) Anmelder: UbiCom Gesellschaft für Telekommunikation mbH, 16540 Hohen Neuendorf (DE)
(72) Erfinder: Pilz, Ulrich, Dr.-Ing., 13465 Berlin (DE)
(74) Vertreter: Heinze, Ekkehard, Dipl.-Phys. Dr.

(57) **Zusammenfassung**

Geräteanordnung zur Fernüberwachung von Körperfunktionen eines medizinisch zu betreuenden Säugetiers, insbesondere Menschen, mit mindestens einem Funktionsmessgerät zur Messung einer körperfunktionsrelevanten physiologischen Größe, einer fern von dem oder jedem Funktionsmessgerät und im Wahrnehmungsbereich eines medizinischen Betreuers angeordneten ersten Anzeigeeinheit zur Anzeige der Messwerte der physiologischen Größe und/oder einer daraus abgeleiteten Funktionskennzeichnung, einer fern von dem oder jedem Funktionsmessgerät angeordneten Eingabeeinheit zur Eingabe von Steuerbefehlen für das Funktionsmessgerät und/oder mindestens ein bei dem Säugetier angeordnetes Therapiegerät und/oder Warnsignalen bzw. Informationen zur Anzeige durch eine zweite Anzeigeeinheit in dessen Wahrnehmungsbereich und einer bidirektionalen öffentlichen Telekommunikationsnetzverbindung, insbesondere Mobilfunkverbindung, mit einem Hin-Kanal zwischen dem Funktionsmessgerät und der ersten Anzeigeeinheit sowie einem Rück-Kanal zwischen der Eingabeeinheit und dem Funktionsmessgerät und/oder Therapiegerät und/oder der zweiten Anzeigeeinheit, wobei für jedes der angeschlossenen Geräte einer an ein Übertragungsprotokoll des Telekommunikationsnetzes angepasste Schnittstelle vorgesehen ist.

## Beschreibung

Die Erfindung betrifft eine Geräteanordnung zur Fernüberwachung von Körperfunktionen eines medizinisch zu betreuenden Menschen oder veterinärmedizinisch zu betreuenden Säugetiers. Zu den realisierbaren Funktionen einer solchen Anordnung gehört auch die sportmedizinische Überwachung sowie gegebenenfalls die Überwachung der Körperfunktionen hochwertiger Tiere in der Hochleistungs-Tierhaltung - der Schwerpunkt der Erfindung liegt jedoch auf der Betreuung menschlicher Patienten mit chronischen Leiden.

Durch die Fortschritte der medizinischen Technik ist es möglich geworden, eine Vielzahl von Krankheiten und Leiden so zu behandeln, dass die betroffenen Personen zwar nicht abschließend geheilt werden, jedoch mit diesen Krankheiten bei guter Lebensqualität leben können. Derartige Leiden (z.B. Diabetes, diverse Herzerkrankungen, Asthma, Krankheiten des rheumatischen Formenkreises, Krebserkrankungen, Morbus Alzheimer, Allergien) erfordern jedoch eine ständige medizinische Betreuung der Patienten, da Medikamente regelmäßig einzunehmen sind und eine ständige diagnostische Überwachung durch entsprechende Messgeräte zu erfolgen hat. Hinzu kommt, dass auch ständig eine fachärztliche Kontrolle der am Patienten erhobenen diagnostischen Daten wie auch eine Betreuung des Patienten durch den Arzt erfolgen muss, um langfristig die Entwicklung des Leidens für den Betroffenen möglichst optimal zu gestalten.

Als weiterer Aspekt kommt hinzu, dass in jüngster Zeit die medizinisch-technischen Möglichkeiten der Behandlung chronisch Kranker (z.B. Sauerstoffbeatmungsanlagen für Asthmakranke) sowie zur Erhebung diagnostischer Daten (z.B. Glukosemessung bei Diabetes) so weit ausgereift sind, dass sie der Patient zuverlässig autonom und ambulant handhaben kann.

Allgemein bekannter Stand der Technik bei Herz- und Kreislauferkrankungen sind Herzkliniken, in denen Infarktpatienten Wochen und Monate zur Beobachtung verbringen. Ergänzt wird das Betreuungssystem durch niedergelassene Ärzte, die EKG anfertigen können und die Patienten mit 24-Stunden-Aufzeichnungsgeräten versehen, so dass ein täglicher Arztbesuch entfallen kann. Bekannt sind auch Systeme, in denen "Herz-Handys" im vom Patienten zu bestimmenden Bedarfsfall auf die Brust gelegt werden, um Daten über das GSM-Netz an eine Arztpraxis zu übertragen.

Stand der Technik bei Asthma-Kranken sind Lungenfunktionsmessgeräte, die der Patient selbständig handhaben kann. Dies ist jedoch - auch in Verbindung mit Arztbesuchen - für eine erfolgreiche Therapie nicht ausreichend, da hierfür die regelmäßige Einnahme der Medikamente (gerade auch in symptomfreien Zeiten, was von vielen Patienten versäumt wird) wie auch situationsbedingte Anpassung des Therapieschemas durch den Arzt erforderlich sind. In vielen Fällen benötigen Patienten mit chronischen Lungenleiden auch spezielle Beatmungsgeräte (erhöhter Sauerstoffgehalt und Filterung von Staub und Pollen), die ebenfalls von Fachkräften gewartet werden müssen.

Aus den Bereichen der Weltraumfahrt und der Sportmedizin ist es auch bekannt, die Körperfunktionen einzelner Personen (Astronauten in einem Raumschiff, Leistungssportler während des Rennens oder Trainings) durch am Körper getragene oder jedenfalls in der Nähe des Untersuchungssubjektes befindliche Messgeräte zu überwachen und die Messergebnisse über eine speziell etablierte Funkstrecke zu einer Auswertungs- und Überwachungszentrale (Bodenstation, Begleitfahrzeug oder Trainingszentrum) zu übertragen. Dort können die Daten - vorteilhafterweise nahezu in Echtzeit - ausgewertet werden. Gegebenenfalls ist auch vorgesehen, den untersuchten Personen per Funk Handlungsanweisungen - etwa zum weiteren Einstellen der körperlichen Belastung oder zu bestimmten Handhabungen - zu übermitteln.

Die letztgenannten Lösungen eignen sich aufgrund des hohen Aufwandes für die Implementierung der Nachrichtenübertragung sowie aufgrund von spezifischen technischen Eigenschaften der hier eingesetzten Übertragungsstrecken nicht für ein großes Gesundheitsüberwachungssystem, in dessen Genuss eine Vielzahl von Patienten, insbesondere chronisch Kranke, kommen kann und das in seiner medizinischen Komponente durch ein weit verzweigtes Netz von Kliniken, Arztpraxen, und anderen medizinischen Betreuungsstellen betrieben werden kann.

Der Erfindung liegt daher die Aufgabe der Bereitstellung eines derartigen, zuverlässig mit relativ geringen Kosten implementier- und betreibbaren Systems zugrunde.

Diese Aufgabe wird durch eine Geräteanordnung mit den Merkmalen des Anspruchs 1 oder des Anspruchs 2 gelöst. Unter einer Vielzahl möglicher Fortbildungen des Erfindungsgedankens ergeben sich wesentliche Gedanken aus den abhängigen Ansprüchen.

Die Erfindung schließt den wesentlichen Gedanken ein, bei dem Subjekt der Untersuchung - nachfolgend auch als "Patient" bezeichnet - befindliche Funktionsmessgeräte über ein öffentliches Telekommunikationsnetz mit im klinischen bzw. ambulanten Bereich befindlichen Anzeigeeinrichtungen bzw. automatischen Überwachungs- und Steuereinrichtungen zu verbinden. Die Nutzung eines öffentlichen Netzes sichert neben erheblichen Kostenvorteilen gegenüber speziell etablierten Funkstrecken eine praktisch unbegrenzte Systemkapazität und außerordentlich hohe Verfügbarkeit der Nachrichtenverbindung und somit überzeugende Zuverlässigkeit der Überwachung. Bei Nutzung eines Mobilfunknetzes, was aus derzeitiger Sicht bevorzugt ist, besteht zudem eine praktisch lückenlose flächendeckende Erreichbarkeit des Patienten, jedenfalls in den Industrieländern.

Weiterhin gehört zur Erfindung der Gedanke, im Rahmen der erwähnten Telekommunikationsnetzverbindung sowohl einen Hin- als auch einen Rück-Kanal zu implementieren, um einerseits am Patienten gewonnene Mess- bzw. Auswertungsergebnisse der Anzeigeeinheit oder Überwachungs- und Steuereinrichtung zuzuführen und andererseits Handlungsanweisungen an den Patienten oder Steuerbefehle an ein bei ihm angeordnetes Mess- bzw. Erfassungsgerät bzw. auch ein zu seiner Fern-Betreuung vorgesehenes Therapiegerät zu übermitteln.

In einer ersten relativ unabhängigen Ausprägung des Erfindungsgedankens schließt die Erfindung den Gedanken der aktiven Einbeziehung von medizinischen Personal in die Fernüberwachung und wahlweise Fernbetreuung des Patienten ein, während gemäß einem zweiten relativ unabhängigen Aspekt der Erfindung eine im wesentlichen automatische Auswertung der am Patienten gewonnenen Messdaten zur automatischen Generierung von Steuerbefehlen bzw. Handlungsanweisungen vorgesehen ist. Beide Aspekte können in Abhängigkeit vom konkreten Entwicklungsstand eines nationalen Gesundheitswesens, in dessen Rahmen die Erfindung eingesetzt wird, und insbesondere von der konkreten Art des Leidens des Patienten und der Maßnahmen zu seiner Therapie die bevorzugte Ausführung darstellen.

In größeren Systemen der erfindungsgemäßen Art dürfte auch eine Kombination beider Varianten vorteilhaft sein, wobei krankheitsabhängig entweder die durch medizinisches Personal ausgelöste oder die automatische Betreuungshandlung das Primat haben wird. Es versteht sich, dass normalerweise zumindest jede automatisierte Betreuungshandlung dokumentiert und zusammen mit den auslösenden Messdaten bzw. dem auslösendem Ereignis zeitlich parallel oder zeitlich versetzt einer ärztlichen Auswertung unterzogen wird.

Aufgrund der obigen Ausführungen versteht es sich des weiteren, dass ein etabliertes System der erfindungsgemäßen Art eine Vielzahl von Funktionsmessgeräten bei einer großen Menge von Patienten einerseits sowie ein ganzes Netz von im medizinischen Bereich angeordneten Anzeigeeinheiten bzw. Überwachungs- und Steuereinrichtungen umfassen wird, wobei auch eine entsprechend große Anzahl von Schnittstellen mit einem Telekommunikationsnetz oder auch mehreren Telekommunikationsnetzen vorgesehen ist. Die Einbeziehung mehrerer unabhängiger Telekommunikationsnetze in das System wird insbesondere bei länderübergreifenden Systemlösungen erforderlich sein, kann aber aus Wettbewerbsund Kostengründen für nationale Systeme eine Rolle spielen. Die beidseits an der Kommunikationsstrecke angeschlossenen Geräte können selbstverständlich sehr unterschiedliche Geräte sein - es ist lediglich die Etablierung eines geeigneten Übertragungsstandards (oder auch mehrerer kompatibler Standards) für deren Kommunikation und das Zusammenwirken erforderlich.

Speziell für Patienten mit Herzleiden ist als Funktionsmessgerät insbesondere eine Pulsfrequenz- bzw. Herzrhythmus-Erfassungseinrichtung (von an sich bekannter Bauart) vorgesehen. Alternativ oder zusätzlich hierzu können Patienten mit Kreislaufkrankheiten - insbesondere chronischen - als Funktionsmessgerät über ein Blutdruckmessgerät und/oder ein Oximeter zur Erfassung des Blutsauerstoffgehaltes verfügen. Daneben sind im Rahmen des vorgeschlagenen Systems zusätzliche Messeinrichtungen einsetzbar, wie Sie bei implantierbaren Herzschrittmachern zur korrekten Messdatenerfassung mitunter zusätzlich zur eigentlichen Herzrhythmus-Erfassungseinrichtung vorgesehen sind - beispielsweise ein Sensor zur Erfassung der Körperlage (stehend/liegend) oder eine Accelerometer zur Erfassung starker körperlicher Aktivitäten.

Diabetiker verfügen vorteilhafterweise über eine eine periodisch selbsttätige Messung des Blutzuckergehaltes ausführende (Glukose-)Messeinrichtung, Patienten mit schwerwiegenden Atemwegserkrankungen über ein - als solches ebenfalls bekanntes und kommerziell verfügbares - Gerät zur Erfassung der Lungenleistung bzw. des Atemrhythmus, und schließlich können Patienten mit teilweise lebensbedrohlichen Nervenkrankheiten eine portable EEG-Aufnahmeeinrichtung zur Erfassung der Hirnströme tragen. Diese Aufzählung ist nicht erschöpfend und soll lediglich wesentliche Anwendungen des vorgeschlagenen Systems und die bei diesem zum Einsatz kommenden Datenerfassungseinrichtungen illustrieren.

In einer praktisch sinnvollen bevorzugten Ausführung umfasst das vorgeschlagene System neben den grundlegenden Komponenten - den Funktionsmessgeräten einerseits und den Anzeigeeinheiten bzw. Überwachungs- und Steuereinheiten andererseits sowie dem diese verbindenden TK-Netz mit Hin- und Rück-Kanal - auch Therapiegeräte und/oder zusätzliche Anzeigeeinheiten auf Patientenseite, um im Ansprechen auf ein eine medizinische Reaktion erforderndes Überwachungsergebnis bei einem Patienten diesem sogleich eine Handlungsanweisung anzeigen bzw. ohne weiteres über sein Therapiegerät eine geeignete Therapie automatisch einleiten zu können.

Für die oben bereits erwähnten Herzpatienten ist das Therapiegerät bevorzugt als (an sich wiederum bekannter) Herzschrittmacher oder aber - falls ihre Herzrhythmusstörung vom hochfrequenten Typ ist und in Fibrillationen umzuschlagen droht - gegebenenfalls als Defibrillator ausgebildet. Diese Herzrhythmus-Korrekturgeräte sind seit längerem in implantierbaren und über kurzreichweitige Telemetriestrecken fernsteuerbaren Ausführungen verfügbar, die auch beim vorgeschlagenem System zur Anwendung kommen können und sollten.

Unter anderem auch für Herzpatienten, aber auch zur Behandlung anderer Krankheiten, die in dringend medikamentös zu behandelnde akute Gefahrenzustände umschlagen können, sind als Therapiegeräte Medikamentendosiergeräte einsetzbar. Auch diese sind bevorzugt fernsteuerbar und natürlich insbesondere (in Gestalt der bekannten implantierbaren Insulinpumpen) vor allem für schwer an Diabetes Erkrankte vorteilhaft einsetzbar. Es bedarf keiner besonderen Erwähnung, dass eine einfachere und kostengünstigere Variante der systemkonformen Realisierung einer medikamentösen Therapie in der Ausgabe einer Handlungsanweisung zur Einnahme eines Medikamentes in Pillen- bzw. Tropfenform oder zu einer Selbstinjektion an den Patienten besteht. Für eine dauerhaft zuverlässige medikamentöse Behandlung, unabhängig von einer Befolgung derartiger Handlungsanweisungen durch den Patienten, haben jedoch die kostenaufwendigeren Medikamentendosiergeräte erhebliche Sicherheitsvorteile.

Für Patienten mit schweren Atemwegserkrankungen sind, insbesondere ebenfalls dann, wenn diese (wie etwa bei Asthmatikern unter plötzlicher allergener Belastung) in akute Gefahrenzustände umschlagen können, als Therapiegerät fernsteuerbare Beatmungsgeräte systemkonform einsetzbar. Diese können insbesondere einen Sauerstoff- oder Aerosolerzeuger umfassen. Auch hier besteht die kostengünstigere Variante speziell bei Asthmatikern in der Ausgabe einer Handlungsanweisung zum Gebrauch eines vom Patienten selbst zu bedienenden entsprechenden Gerätes - aber auch hier haben über den Rück-Kanal der TK-Verbindung zentral steuerbare Geräte gegebenenfalls Sicherheitsvorteile.

Neben den erwähnten Geräten, die als Diagnose- bzw. Therapiegeräte im engeren Sinne anzusprechen sind, umfasst das System in einer vorteilhaften Ausgestaltung patientenseitig weiterhin eine Bildaufnahmeeinrichtung sowie "zentral" (d.h. entfernt von der Bildaufnahmeeinrichtung) eine Videoempfangs- und Anzeigeeinrichtung zur Anzeige von beim Patienten aufgenommenen Bildern für das medizinische Personal zur erleichternden Beurteilung des Patientenzustandes. Bei schweren chronischen dermatologischen Leiden kann diese Bildaufnahmeinrichtung (Videokamera) auch an die Stelle des Funktionsmessgerätes treten, und das von ihr gelieferte Bild wird analog zu Messdaten bei anderen Krankheiten ausgewertet.

Die den einzelnen Systemkomponenten zugeordneten Schnittstellen zum Anschluss an das TK-Netz sind in einer bevorzugten Ausführung als Mobilfunk-Endgeräte mit geeigneten Datenein- bzw. -ausgängen ausgebildet. Unter dem Begriff "Mobilfunk-Endgerät" sollen im Rahmen der Erfindung neben normalen Mobiltelefonen auch Sende-/Empfangsteile mit reduzierter Funktionalität (beispielsweise ohne Mittel zur Sprachübertragung) zu verstehen sein, ebenso wie Handheld-PCs bzw. PDAs (Personal Digital Assistants) mit Mobilfunkteil als besonders komfortable und variable Ausführung.

In für andere Anwendungen bekannter Weise können Hin- und Rück-Kanal zur Datenübertragung im SMS oder EMS-Format oder unter Nutzung des WAP-Standards (speziell per WAP-Push) ausgebildet sein. Im Hin-Kanal erfolgt die Sendeaktivierung bevorzugt automatisch über das Funktionsmessgerät bzw. die Bildaufnahmeeinrichtung, während sie im Rück-Kanal durch das die Ergebnisse auswertende medizinische Personal "manuell" oder durch die Überwachungs- und Steuereinheit in der Betreuungszentrale automatisch erfolgt. Neben den genannten Möglichkeiten besteht auch die sinnvolle Möglichkeit der Realisierung von Hin- und Rück-Kanal als permanente, paketorientierte Verbindung ("Standleitung") gemäß dem GPRS-Standard im Rahmen eines GSM-Netzes oder gemäß dem künftigen UMTS-Standard.

Zur Datenhaltung ist ärztlicherseits - aus Kostengründen bevorzugt zentral - eine Datenbank zur Speicherung von relevanten übertragenen Messdaten bzw. Informationskennzeichnung vorgesehen, und dieser ist insbesondere ein Datenbank-Managementsystem zur (bevorzugt wiederum zentralen) Verwaltung dieser Daten für die medizinischen Betreuungseinrichtungen zugeordnet. Das Datenhaltungssystem ist aus derzeitiger Sicht bevorzugt über ein Internetportal aufgrund einer entsprechenden Autorisierung zugänglich. Mit abgestuften Autorisierungsprüfungen und Zugriffssteuerungen kann sowohl dem autorisierten medizinischen Personal (etwa dem Hausarzt) als auch den Patienten ein einfacher Zugriff auf gespeicherte Daten von beliebigen Endgeräten aus gewährt werden.

Die erwähnten Funktionsmessgeräte bzw. Therapiegeräte können über einen Hin- bzw. Rück-Kanal an das System angeschlossen sein, welcher eine drahtlose Kurzstreckenverbindung zu einer zugeordneten Schnittstelle des TK-Netzes umfasst. Ebenso kann die erwähnte patientenseitige Bildaufnahmeeinrichtung (Videokamera bzw. Webcam) über eine solche drahtlose Verbindung angeschlossen sein, bei der es sich aus derzeitiger Sicht zweckmäßigerweise um eine Wireless-LAN-, Bluetooth- oder DECT-Verbindung handeln wird. Unter Kostengesichtspunkten erscheinen hierbei insbesondere die Bluetooth- und die DECT-Verbindung als vorteilhaft, wobei nach vorläufigen Erkenntnissen der Bluetooth-Standard noch Zuverlässigkeitsvorteile bietet.

Durch die Anwendung der Erfindung kann ein Patient auch zuhause oder auf Reisen annähernd die gleiche Behandlungsqualität erhalten, wie er sie in der stationären Aufnahme in einer Klinik erwarten könnte. Über die räumliche Entfernung hinweg können erstens die personenbezogenen medizinischen Daten übermittelt und fachärztlich bewertet werden. Zweitens ist die kontrollierte Einhaltung der Medikation gewährleistet. Drittens ist die direkte, videogestützte Kontaktaufnahme zwischen Patient/Pflegepersonal und Arzt gegeben. Viertens können medizinische Geräte ferngesteuert und ferngewartet werden. Fünftens hat der Patient über das Internetportal jederzeit Zugriff auf seine persönlichen Daten, Hinweise vom Arzt, Wartungstermine der von ihm benutzten Geräte, etc.

In der Durchführung medizinischer Studien aber auch bei Verordnungen ist die "compliance" der Probanden, d.h. der Grad der Genauigkeit, mit der sie sich an die Vorschriften zur Einnahme der Medikamente und Anwendungen halten, von entscheidender Bedeutung. Hier trägt die Erfindung dazu bei, die Daten zuverlässig aufzuzeichnen, die Daten zu verwalten und die Daten auf Richtigkeit zu prüfen. Die Erfindung ermöglicht auf einfache Art und Weise die Anwendung von Geräten für ein objektives Messen der Patienten-"compliance".

Vorteile und Zweckmäßigkeiten der Erfindung ergeben sich im übrigen aus den Unteransprüchen sowie der nachfolgenden Beschreibung bevorzugter Ausführungsbeispiele anhand der Figuren. Von diesen zeigen:
- Fig. 1: eine Prinzipskizze einer Anordnung gemäß einer ersten Ausführungsform der Erfindung,
- Fig. 2: eine Prinzipskizze einer Anordnung gemäß einer zweiten Ausführungsform der Erfindung,
- Fig. 3: eine Prinzipskizze einer Anordnung gemäß einer dritten Ausführungsform der Erfindung und
- Fig. 4: eine Prinzipskizze einer Anordnung gemäß einer vierten Ausführungsform der Erfindung.

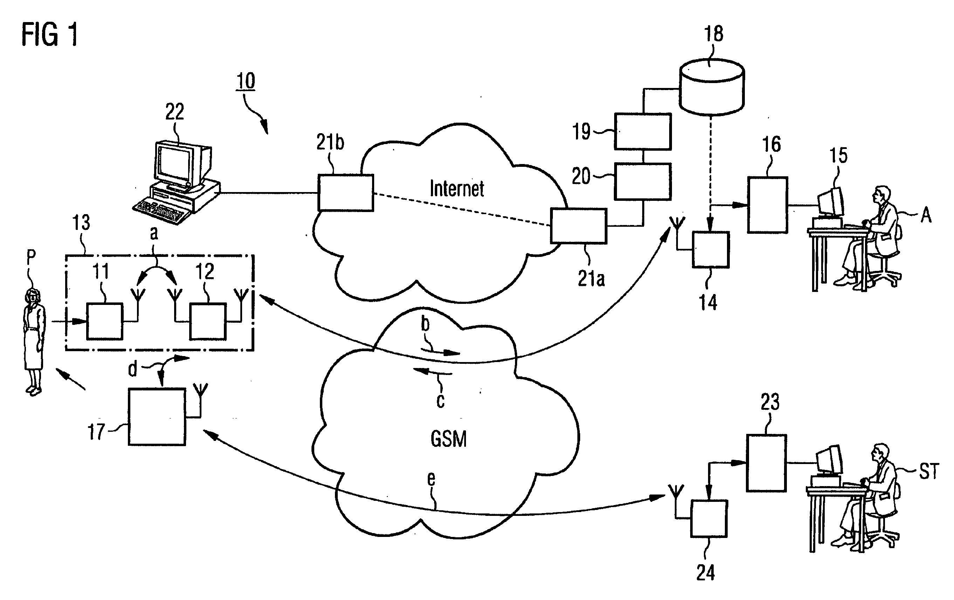

Fig. 1 zeigt als erste Ausführungsform der Erfindung eine Geräteanordnung 10 zur Betreuung eines sich entfernt aufhaltenden Asthmakranken P durch ein lungenfachärztliches Zentrum. Der Patient P verfügt über ein einfach aufgebautes und von ihm selbst problemlos zu handhabendes Lungenfunktions-Diagnosegerät 11 mit Bluetooth-Karte, welches am Körper zu tragen ist und über eine Kurzstrecken-Funkverbindung a nach dem Bluetooth-Standard in permanenter Verbindung mit einem in gleicher Weise ausgestatteten Mobilfunk-Endgerät 12 steht.

Es handelt sich hierbei beispielsweise um ein GSM-Mobiltelefon mit Dateneingang als Schnittstelle zu einem (hier nur schematisch dargestellten) Mobilfunknetz GSM, über das im Bedarfsfall eine langreichweitige Funkverbindung b zu einem weiteren Mobilfunk-Endgerät 14 bei einem Arzt A des lungenfachärztlichen Zentrums aufgebaut wird. Das Diagnosegerät 11 und patientenseitige Mobilfunk-Endgerät 12 können zusammen auch als Patientenzustands-Überwachungseinheit 13 bezeichnet werden.

Im Lungenfunktions-Diagnosegerät 11 ist mindestens ein Schwellwert einer die Lungenfunktion bzw. Atemtätigkeit kennzeichnenden Größe, beispielsweise der Atemfrequenz und/oder des Atemvolumens, gespeichert, und das Gerät ist zur Erfassung entsprechender aktueller Werte und zum Vergleich derselben mit dem Schwellwert ausgebildet. Bei signifikanter Unterschreitung des Schwellwertes oder der Schwellwerte übermittelt die Vergleichereinheit selbsttätig über die Bluetooth-Strecke a ein Alarmsignal an das Mobiltelefon 12, welches dort ebenfalls selbsttätig die Absendung einer Alarmmeldung über die Mobilfunkstrecke b an das klinikseitige Mobiltelefon 14 und eine Alarmmeldung auf einer an dieses angeschlossenen Anzeigeeinheit 15 (einem PC-Bildschirm) auslöst.

Über den zugehörigen PC 16 als Eingabeeinheit kann der Arzt A daraufhin einen Fernsteuerbefehl zur Aktivierung eines ständig in der Nähe des Patienten befindlichen oder von diesem getragenen Therapiegerätes (Aerosolapplikators) 17 geben, der über einen durch sein Mobiltelefon 14 und das patientenseitige Mobiltelefon 12 etablierten Rück-Kanal und eine zweite Bluetooth-Strecke zwischen dem Mobiltelefon 12 und dem Aerosolapplikator 17 übertragen wird und automatisch eine vorbestimmte Therapie durch Aerosolabgabe beim Patienten auslöst. Die Therapie erfolgt also hier unabhängig vom Patientenzustand und dessen aktuellen Handlungsmöglichkeiten und daher zuverlässig auch bei einem schweren akuten Asthmaanfall.

Die bei Erfassung einer Unregelmäßigkeit in der Atemtätigkeit des Patienten übermittelten Lungenfunktions-Messdaten bzw. das eine Unregelmäßigkeit kennzeichnende Alarmsignal werden - mit einem Zeitstempel versehen - neben der Anzeigeeinheit 15 auch einem zentralen Patientendatenspeicher 18 des fachärztlichen Zentrums zugeführt und dort patientenbezogen gespeichert. Die Datenverwaltung erfolgt durch eine zugeordnete Datenmanagementeinheit 19, der eine Autorisierungsprüfstufe 20 zur Prüfung der Autorisierung von externen Datenzugriffen zugeordnet ist. Über eine klinikseitige Datennetz-Schnittstelle 21a ist der Patientendatenspeicher 18 an das Internet angebunden, und über sein Mobiltelefon 12 oder einen patientenseitigen PC 22 und eine patientenseitige Datennetz-Schnittstelle 21b kann der Patient nach Eingabe eines entsprechenden Autorisierungscodes auf seine eigenen Daten aus der Vergangenheit zugreifen.

Der Aerosolapplikator 17 empfängt über seine Bluetooth-Verbindung c zum patientenseitigen Mobiltelefon 12 nicht nur im Falle des Auftretens eines Therapiebedarfs die entsprechenden Steuersignale aus dem medizinischen Zentrum, sondern auch in regelmäßigen Wartungsintervallen Funktionskontrollsignale, die entfernt von einem Servicetechniker ST über dessen PC 23 eingegeben und über ein technikerseitiges Mobilfunk-Endgerät 24 und eine gesonderte Verbindung e im Mobilfunknetz GSM übertragen werden. Der PC 23 des Servicetechnikers ST hat hier also die Funktion einer Fernüberwachungs- und Fernwartungseinrichtung, und die drahtlose Anbindung sichert auch hier die Möglichkeit, die erforderlichen Aktivitäten unabhängig vom aktuellen Aufenthaltsort des Patienten durchzuführen.

Service- und ggf. Reparaturbesuche des Servicetechnikers ST beim Patienten P werden durch diese Fernwartungsmöglichkeiten nicht grundsätzlich überflüssig, ihre Häufigkeit kann aber verringert und die Effizienz erhöht werden. Es besteht im übrigen die Möglichkeit, das der Servicetechniker zur Vereinbarung von vor Ort durchzuführenden Wartungsarbeiten über die Mobilfunkverbindung oder alternativ über das Internet, direkt Verbindung mit dem Patienten aufnimmt - die entsprechenden Möglichkeiten sind aber hier zur Erhaltung der Klarheit der Darstellung nicht eingezeichnet.

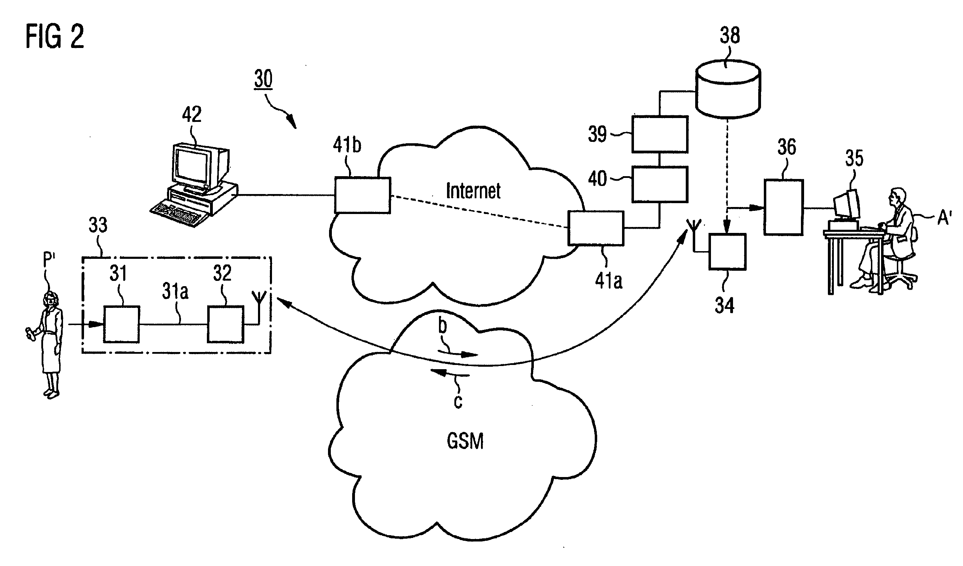

In Fig. 2 ist als weiteres Beispiel eines erfindungsgemäßen Systems eine Geräteanordnung 30 zur Fernbetreuung von Bluthochdruckpatienten P' durch einen Arzt A' in einer Arztpraxis skizzenartig dargestellt. Funktionell mit Komponenten der Anordnung nach Fig. 1 im wesentlichen übereinstimmende Komponenten sind mit an die dortige Darstellung angelehnten Bezugsziffern bezeichnet und werden - sofern es bei der hier vorliegenden Anordnung nicht Besonderheiten zu beachten gibt - nicht nochmals erläutert.

Die Geräteanordnung 30 umfasst ein vom Patienten zu tragendes und selbst zu bedienendes Blutdruckmessgerät 31, welches über ein Datenkabel 31a mit einem Mobiltelefon 32 des Patienten zu verbinden ist. Das Blutdruckmessgerät 31 und das Mobiltelefon 32 bilden wieder (wie beim ersten Beispiel) eine Patienten-Überwachungseinheit 33.

Der Arzt A' verfügt über die gleichen Systemkomponenten, die im lungenfachärztlichen Zentrum beim ersten Beispiel zur Verfügung standen, und auch die Signalübertragung von als irregulär anzusehenden Blutdruckmesswerten zu ihm hin ist gleichartig wie beim ersten Beispiel. Anders als dort, verfügt der Patient hier aber nicht über ein Therapiegerät, sondern erhält vom Arzt beim Auftreten unzulässig hoher oder niedriger Blutdruckwerte eine konkrete Medikationsanweisung über den Rück-Kanal e der langreichweitigen Mobilfunkstrecke zwischen dem arztseitigen Mobiltelefon 34 und dem patientenseitigen Mobiltelefon 32. Das patientenseitige Mobiltelefon 32 dient bei dieser Signalübermittlung zugleich als Anzeigeeinheit, über das der Patient per Sprachausgabe oder per Displayanzeige einen Hinweis auf die Einnahme blutdrucksenkender Mittel erhält.

Hinsichtlich der Verfügbarkeit einer Patientendatenbasis besteht wieder Übereinstimmung mit dem ersten Beispiel nach Fig. 1, während bei dem hier eingesetzten, in großen Stückzahlen hergestellten und über lange Zeit zuverlässig arbeitenden Diagnosegerät und angesichts des Fehlens eines Therapiegerätes auf Möglichkeiten zur Fernwartung verzichtet werden kann.

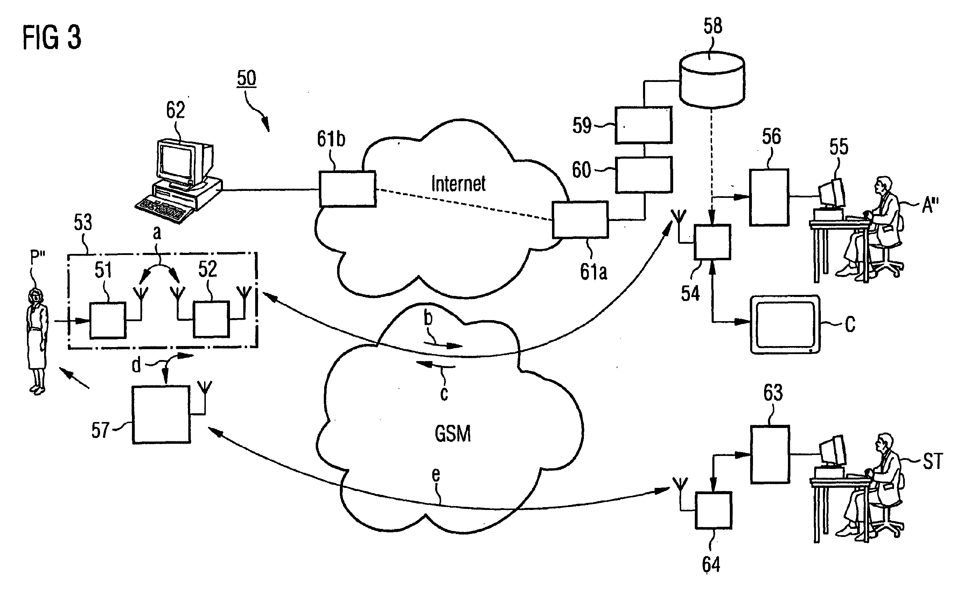

Fig. 3 zeigt - ebenfalls schematisch - eine dritte Geräteanordnung 50 zur Ferndiagnose und -therapie eines unter chronischer Diabetes leidenden Patienten durch eine zentrale Therapiesteuereinheit C unter Überwachung eines Diabetesarztes A" in einem Diabeteszentrum. Der grundlegende Aufbau dieser Anordnung entspricht dem des ersten Beispiels nach Fig. 1, so dass auch hier weitgehend gleichwirkende Komponenten mit an Fig. 1 angelehnten Bezugsziffern bezeichnet sind und hier nicht nochmals beschrieben werden.

Bei dem Diagnosegerät handelt es sich hier um ein implantiertes Glucometer 51 und bei dem Therapiegerät um eine ebenfalls implantierte, fernsteuerbare Insulinpumpe 57. Die Ausgestaltung von Hin- und Rück-Kanal einer Mobilfunkverbindung zwischen diesen patientenseitigen Geräten und dem Diabeteszentrum entspricht grundsätzlich der nach Fig. 1 - mit der Ausnahme, dass jetzt neben der Verbindung zu einem PC 56 des Diabetesarztes auch eine direkte Verbindung zu der Therapiesteuereinheit C aufgebaut wird, sobald durch das Glucometer bedenkliche Blutzuckerwerte erfasst werden.

Das Therapiesteuergerät C gibt, ohne dass ein Bedieneingriff des Arztes erforderlich wäre (der natürlich zulässig ist), in Reaktion auf eine Auswertung der übermittelten Blutzuckerwerte einen Steuerbefehl für die Insulinpumpe aus, der über den Rück-Kanal der Mobilfunkverbindung zur Insulinpumpe übertragen wird und diese in vorbestimmter Weise aktiviert bzw. den Insulinausstoß ändert.

Bestimmte Überprüfungs- bzw. Wartungsfunktionen für das Glucometer bzw. die Insulinpumpe sind analog zum System nach Fig. 1 als Fernwartung implementiert, und auch die Patientendatenhaltung in einer Datenbasis sowie deren Verwaltung ist entsprechend organisiert.

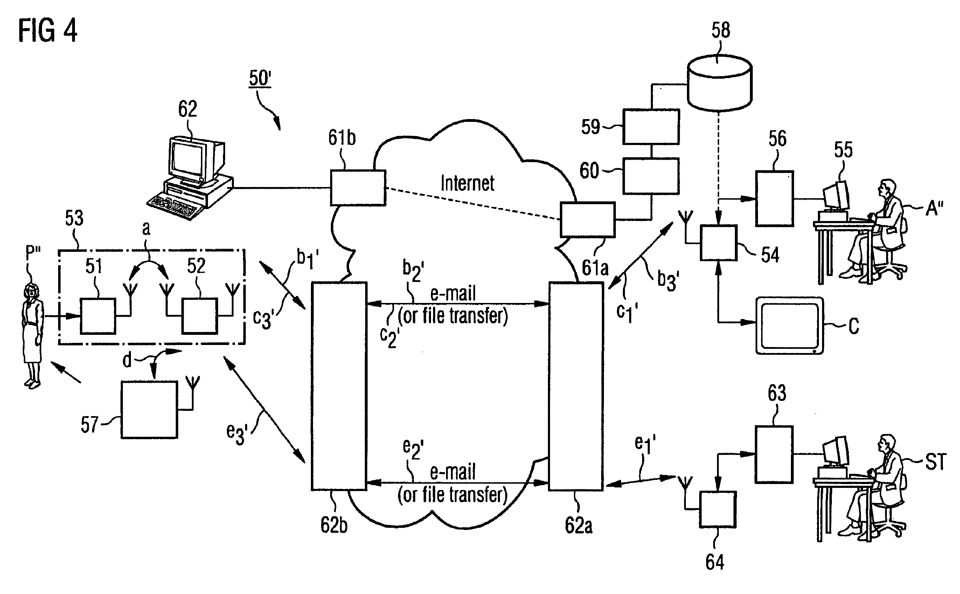

Fig. 4 zeigt - unter Beibehaltung der in Fig. 3 vergebenen Bezugsziffern der dritten Ausführungsform - eine vierte Ausführungsform, die gegenüber der dritten hinsichtlich der Netzstruktur für die relevanten Informationsübertragungsvorgänge modifiziert ist. Die Gesamtanordnung wird mit der Bezugsziffer 50' bezeichnet und unterscheidet sich von der dritten Ausführungsform dadurch, dass für sämtliche Nachrichtenübertragungen das Internet genutzt wird.

Die langreichweitigen Nachrichtenübertragungen zwischen der Patientenzustands-Überwachungseinheit 53 und dem klinik- bzw. arztseitigen Mobilfunk-Endgerät 54 sowie zwischen diesem und der Patientenzustands-Überwachungseinheit in Gegenrichtung sind hier in jeweils drei Abschnitte b1' bis b3' bzw. c1' bis c3' unterteilt. Der erste und letzte Abschnitt ist jeweils durch eine Mobilfunkstrecke zwischen dem jeweiligen Endgerät und einem Gateway 62a bzw. 62b zum Internet gebildet, während der mittlere Abschnitt durch die Übersendung einer E-Mail oder einen Filetransfer innerhalb des Datennetzes gebildet ist. Die Gateways 62a, 62b führen in an sich bekannter und von Providern als Dienstleistung bereitgestellter Weise eine SMS/E-Mail- bzw. E-Mail/SMS-Konversion durch.

In gleicher Weise ist bei dieser Ausführungsform die Datenübertragung zwischen dem Mobilfunk-Endgerät 64 des Servicetechnikers ST und der Insulinpumpe 57 (über das Mobilfunkteil 52) organisiert. Im übrigen kann für die in Rede stehenden Datenübertragungen auch eine andere Kombination gängiger Übertragungsformate mit entsprechender Wandlung vorgesehen sein, etwa eine Kombination von SMS- und Faxübertragung oder von E-Mail- und Faxübertragung.

Die Ausführung der Erfindung ist nicht auf deren oben hervorgehobene Aspekte sowie die bevorzugten Ausführungsbeispiele beschränkt, sondern ebenso in einer Vielzahl von Abwandlungen möglich, die im Rahmen fachgemäßen Handelns liegen.

### Bezugszeichenliste

- 10; 30; 50: Geräteanordnung
- 11: Lungenfunktions-Diagnosegerät
- 12, 14; 24; 32, 34; 52, 54; 64: Mobilfunk-Endgerät
- 13; 33; 53: Patientenzustands-Überwachungseinheit
- 15; 35; 55: Anzeigeeinheit (PC-Bildschirm)
- 16; 22, 23; 36; 42; 62, 63: PC
- 17: Aerosolapplikator
- 18; 38; 58: Patientendatenspeicher
- 19; 39; 59: Datenmanagementeinheit
- 20; 40; 60: Autorisierungsstufe
- 21a, 21b; 41a, 41b; 61a, 61b: Datennetz-Schnittstelle
- 31: Blutdruckmessgerät
- 31a: Datenkabel
- 51: Glucometer
- 57: Insulinpumpe
- 65a, 65b: Gateway

- a: kurzreichweitige Funkstrecke (Bluetooth-Strecke)
- A; A'; A'': Arzt
- b; c; d; e; b1' bis b3', c1' bis c3', e1' bis e3': langreichweitige Funkstrecke (Mobilfunkverbindung)
- B: langreichweitige Funkstrecke
- C: Therapiesteuergerät
- GSM: Mobilfunknetz
- P; P'; P": Patient
- ST: Servicetechniker

## Patentansprüche

1. Geräteanordnung zur Fernüberwachung von Körperfunktionen eines medizinisch zu betreuenden Säugetiers, insbesondere Menschen, mit
- mindestens einem Funktionsmessgerät zur Messung einer körperfunktionsrelevanten physiologischen Größe,
- einer fern von dem oder jedem Funktionsmessgerät und im Wahrnehmungsbereich eines medizinischen Betreuers angeordneten ersten Anzeigeeinheit zur Anzeige der Messwerte der physiologischen Größe und/oder einer daraus abgeleiteten Funktionskennzeichnung,
- einer fern von dem oder jedem Funktionsmessgerät angeordneten Eingabeeinheit zur Eingabe von Steuerbefehlen für das Funktionsmessgerät und/oder mindestens ein bei dem Säugetier angeordnetes Therapiegerät und/oder Warnsignalen bzw. Informationen zur Anzeige durch eine zweite Anzeigeeinheit in dessen Wahrnehmungsbereich und
- einer bidirektionalen öffentlichen Telekommunikationsnetzverbindung, insbesondere Mobilfunkverbindung, mit einem Hin-Kanal zwischen dem Funktionsmessgerät und der ersten Anzeigeeinheit sowie einem Rück-Kanal zwischen der Eingabeeinheit und dem Funktionsmessgerät und/oder Therapiegerät und/oder der zweiten Anzeigeeinheit, wobei für jedes der angeschlossenen Geräte einer an ein Übertragungsprotokoll des Telekommunikationsnetzes angepasste Schnittstelle vorgesehen ist.

2. Geräteanordnung zur Fernüberwachung von Körperfunktionen eines medizinisch zu betreuenden Säugetiers, insbesondere Menschen, mit
- mindestens einem Funktionsmessgerät zur Messung einer körperfunktionsrelevanten physiologischen Größe,
- einer fern von dem oder jedem Funktionsmessgerät angeordneten automatischen Überwachungs- und Steuereinheit zur Auswertung der Messwerte der physiologischen Größe und/oder einer daraus abgeleiteten Funktionskennzeichnung und zur Erzeugung von Steuerbefehlen für das Funktionsmessgerät und/oder mindestens ein bei dem Säugetier angeordnetes Therapiegerät und/oder eine Anzeigeeinheit in dessen Wahrnehmungsbereich im Ansprechen auf das Auswertungsergebnis und
- einer bidirektionalen öffentlichen Telekommunikationsnetzverbindung, insbesondere Mobilfunkverbindung, mit einem Hin-Kanal zwischen dem Funktionsmessgerät und der Überwachungs- und Steuereinheit sowie einem Rück-Kanal zwischen dieser und dem Funktionsmessgerät und/oder Therapiegerät und/oder der Anzeigeeinheit, wobei für jedes der angeschlossenen Geräte eine an ein Übertragungsprotokoll des Telekommunikationsnetzes angepasste Schnittstelle vorgesehen ist (im Ansprechen auf das Auswertungsergebnis).

3. Geräteanordnung nach Anspruch 1 oder 2,
**gekennzeichnet durch**
eine bei dem Säugetier angeordnete Videokamera und eine entfernt von der Videokamera angeordnete, mit dieser **durch** den Hin-Kanal der öffentlichen Telekommunikationsnetzverbindung verbundene Videoempfangs- und -anzeigeeinrichtung zum Empfang und zur Darstellung von mittels der Videokamera aufgenommenen Bildern des Säugetiers.

4. Geräteanordnung nach einem der vorgehenden Ansprüche,
**gekennzeichnet durch**
eine bei dem Säugetier angeordnete zweite Anzeigeeinheit zur Anzeige von an der entfernt angeordneten Eingabeeinheit eingegebenen oder **durch** die Überwachungs- und Steuereinheit erzeugten Warnsignalen bzw. Informationen.

5. Geräteanordnung nach einem der vorangehenden Ansprüche,
**gekennzeichnet durch**
mindestens ein bei dem Säugetier angeordnetes Therapiegerät zur Ausgabe einer Therapie im Ansprechen auf einen bei der Eingabeeinheit eingegebenen oder **durch** die Überwachungs- und Steuereinheit erzeugten Steuerbefehl.

6. Geräteanordnung nach einem der vorangehenden Ansprüche,
**gekennzeichnet durch**
eine drahtlose Kurzstreckenverbindung zwischen dem Funktionsmessgerät und wahlweise der Videokamera und/oder dem Therapiegerät und/oder der zweiten Anzeigeeinheit und einer diesem bzw. dieser zugeordneten Schnittstelle des Telekommunikationsnetzes, insbesondere nach Bluetooth-, Wireless LAN- oder DECT-Standard.

7. Geräteanordnung nach einem der vorangehenden Ansprüche,
**dadurch gekennzeichnet, dass**
das oder ein Funktionsmessgerät als Pulsmesser zur Erfassung der Pulsfrequenz oder als Herzrhythmus-Erfassungseinrichtung ausgebildet ist.

8. Geräteanordnung nach einem der vorangehenden Ansprüche,
**dadurch gekennzeichnet, dass**
das oder ein Funktionsmessgerät als Blutdruckmesser ausgebildet ist.

9. Geräteanordnung nach einem der vorangehenden Ansprüche,
**dadurch gekennzeichnet, dass**
das oder ein Funktionsmessgerät als Oximetereinrichtung zur Erfassung des Blutsauerstoffgehaltes ausgebildet ist.

10. Geräteanordnung nach einem der vorangehenden Ansprüche,
**dadurch gekennzeichnet, dass**
das oder ein Funktionsmessgerät als Glukosemesseinrichtung zur Erfassung des Blutzuckergehaltes ausgebildet ist.

11. Geräteanordnung nach einem der vorangehenden Ansprüche,
**dadurch gekennzeichnet, dass**
das oder ein Funktionsmessgerät als Erfassungsgerät zur Erfassung des Atemrhythmus und/oder der Lungenleistung ausgebildet ist.

12. Geräteanordnung nach einem der vorangehenden Ansprüche,
**dadurch gekennzeichnet, dass**
das oder ein Funktionsmessgerät als EEG-Aufnahmeeinrichtung zur Erfassung der Hirnströme ausgebildet ist.

13. Geräteanordnung nach einem der vorangehenden Ansprüche,
**dadurch gekennzeichnet, dass**
das oder ein Funktionsmessgerät eine Auswertungseinrichtung zur Gewinnung einer Funktionskennzeichnung aus den erfassten Messwerten aufweist.

14. Geräteanordnung nach einem der Ansprüche 5 - 13,
**dadurch gekennzeichnet, dass**
das oder ein Therapiegerät als fernsteuerbarer, insbesondere implantierter, Herzschrittmacher ausgebildet ist.

15. Geräteanordnung nach einem der Ansprüche 5 - 13,
**dadurch gekennzeichnet, dass**
das oder ein Therapiegerät als fernsteuerbarer, insbesondere implantierter, Defibrillator ausgebildet ist.

16. Geräteanordnung nach einem der Ansprüche 5 - 13,
**dadurch gekennzeichnet, dass**
das oder ein Therapiegerät als fernsteuerbares Beatmungsgerät, insbesondere mit Sauerstoff- oder Aerosolerzeuger, ausgebildet ist.

17. Geräteanordnung nach einem der Ansprüche 5 - 13,
**dadurch gekennzeichnet, dass**
das oder ein Therapiegerät als fernsteuerbares Medikamentendosiergerät, insbesondere implantierte Insulinpumpe, ausgebildet ist.

18. Geräteanordnung nach einem der vorangehenden Ansprüche,
**dadurch gekennzeichnet, dass**
die Schnittstelle des Funktionsmessgerätes ein erstes Mobilfunk-Endgerät mit Dateneingang aufweist.

19. Geräteanordnung nach einem der Ansprüche 4 - 19,
**dadurch gekennzeichnet, dass**
die zweite Anzeigeeinheit mit integrierter Schnittstelle als erstes Mobilfunk-Endgerät ausgebildet ist.

20. Geräteanordnung nach einem der vorangehenden Ansprüche,
**dadurch gekennzeichnet, dass**
die Schnittstelle der ersten Anzeigeeinheit sowie wahlweise die Schnittstelle des Therapiegerätes ein zweites Mobilfunk-Endgerät mit Datenausgang aufweist.

21. Geräteanordnung nach Anspruch 20,
**dadurch gekennzeichnet, dass**
das zweite Mobilfunk-Endgerät als Handheld-PC mit integriertem Mobilfunkteil ausgebildet ist.

22. Geräteanordnung nach einem der vorangehenden Ansprüche,
**dadurch gekennzeichnet, dass**
der Hin-Kanal zur Datenübertragung per SMS oder EMS oder WAP-Push mit automatischer Sendeaktivierung über das Funktionsmessgerät ausgebildet ist.

23. Geräteanordnung nach einem der vorangehenden Ansprüche,
**dadurch gekennzeichnet, dass**
der Rück-Kanal zur Datenübertragung per SMS oder EMS oder WAP-Push mit manueller Sendesteuerung oder Sendesteuerung durch die Überwachungs- und Steuereinheit im Ansprechen auf das Auswertungsergebnis ausgebildet ist.

24. Geräteanordnung nach einem der Ansprüche 1 - 22,
**dadurch gekennzeichnet, dass**
Hin- und Rück-Kanal als permanente paketorientierte Datenverbindungen gemäß GPRS oder UMTS ausgebildet sind.

25. Geräteanordnung nach einem der vorangehenden Ansprüche,
**gekennzeichnet durch**
eine der ersten Anzeigeeinheit bzw. Überwachungs- und Steuereinheit zugeordnete Datenbasis zur patientenbezogenen Speicherung von übertragenen Messdaten bzw. Funktionskennzeichnungen und ein Datenbank-Managementsystem zu deren Verwaltung, einschließlich Ausgabe an autorisierte Nutzer.

26. Geräteanordnung nach Anspruch 25,
**dadurch gekennzeichnet, dass**
die Datenbasis bzw. das Datenbank-Managementsystem über ein Internetportal mit einem öffentlichen Daten- bzw. Telekommunikationsnetz, insbesondere dem zur Übertragung der Messwerte bzw. der Funktionskennzeichnungen benutzten Telekommunikationsnetz, verbunden ist.

27. Geräteanordnung nach Anspruch 26,
**dadurch gekennzeichnet, dass**
dem Internetportal Prüfmittel zur Autorisierungsprüfung eines potentiellen Nutzers und mit dem Prüfmitteln verbunden Zugriffssteuermittel zur Gewährung eines Zugriffs auf die Datenbasis im Ansprechen auf ein positives Ergebnis der Autorisierungsprüfung zugeordnet sind.

28. Geräteanordnung nach einem der vorangehenden Ansprüche,
**dadurch gekennzeichnet, dass**
die bidirektionale öffentliche Telekommunikationsnetzverbindung einen Übertragungsabschnitt in einem IP-Netz aufweist und an den Grenzen dieses Übertragungsabschnittes Gateways zur Herstellung einer Verbindung mit dem öffentlichen Telekommunikationsnetz und zur Übertragungsformatwandlung vorgesehen sind.

29. Geräteanordnung nach Anspruch 28,
**dadurch gekennzeichnet, dass**
die Gateways zur SMS/E-Mail- bzw. E-Mail/SMS-Wandlung oder zur SMS/Fax- bzw. Fax/SMS-Wandlung oder zur E-Mail/Fax- bzw. Fax/E-Mail-Wandlung ausgebildet sind.
